Europäisches Patentamt
European Patent Office
Office européen des brevets

(19)

(11) Publication number: **0 300 073 A1**

(12) # EUROPEAN PATENT APPLICATION

(21) Application number: 87110621.7

(51) Int. Cl.4: A61K 31/71 , A61K 31/56

(22) Date of filing: 22.07.87

(43) Date of publication of application: 25.01.89 Bulletin 89/04

(84) Designated Contracting States: AT BE CH DE ES FR GB GR IT LI LU NL SE

(71) Applicant: LEO PHARMACEUTICAL PRODUCTS LTD. A/S (LOVENS KEMISKE FABRIK PRODUKTIONSAKTIESELSKAB)
Industriparken 55
DK-2750 Ballerup(DK)

(72) Inventor: Faber, Johan Vigo
Frederiksborggade 43
DK-1360 Copenhagen K(DK)
Inventor: Godtfredsen, Wagn Ole
Noerrevaenget 34
DK-3500 Vaerloese(DK)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40(DE)

(54) **Use of fusidic acid in the treatment of aids-related complex and full-blown aids.**

(57) Fusidic acid and its pharmaceutically acceptable salts are useful for the prophylactic and or therapeutic treatment of AIDS-related complex and full-blown AIDS. Pharmaceutical compositions for this indication and a method for their preparation are disclosed.

EP 0 300 073 A1

## USE OF FUSIDIC ACID IN THE TREATMENT OF AIDS-RELATED COMPLEX AND FULL-BLOWN AIDS

The present invention relates to the use of fusidic acid and its pharmaceutically acceptable salts in the treatment of AIDS-related complex and full-blown AIDS. In particular, the present invention relates to the use of fusidic acid and its pharmaceutically acceptable salts for the manufacture of a medicament for the prophylactic and/or therapeutic treatment of AIDS-related complex and full-blown AIDS. Furthermore, the present invention is directed to a method for the prophylactic and/or therapeutic treatment of patients suffering from or being in risk of contracting AIDS-related complex or full-blown AIDS, which comprises administering to said patients fusidic acid or a pharmaceutically acceptable salt thereof.

Fusidic acid is a narrow spectrum antibiotic having the following structure:

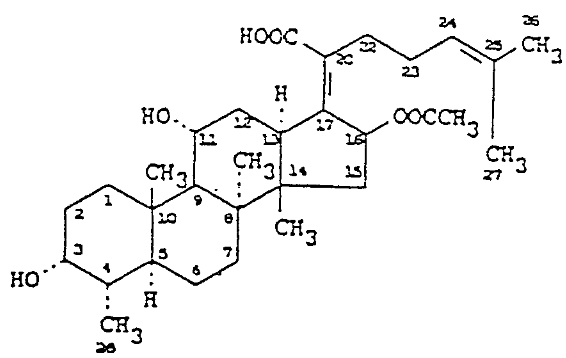

It is formed by fermentation of the fungus Fusidium coccineum and isolated in the form of the free acid or a salt (see, for example, US-A-3 072 531 and GB-A-930 786; W. von Daehne, S. Jahnsen, I. Kirk, R. Larson, H. Lorck: Fusidic Acid: Properties, Biosynthesis and Fermentation in "Biotechnology of Industrial Antibiotics", ED. E.J. Vandamme, New York, Marcel Decker, 1984, pages 427 to 449; and W.O. Godtfredsen: Fusidic acid and some related antibiotics, Dissertation 1967, University of Copenhagen.

Since its introduction in 1962, fusidic acid has been used extensively both systemically and topically in the treatment of bacterial infections, in particular, staphylococcal infections. It has only weak activity against gram negative bacilli and fungi. At usual therapeutic doses (up to 3 g/day) it is essentially non-toxic. In order to control the emergence of fusidic acid-resistant mutants and/or to synergistically potentiate its antibacterial activity, fusidic acid is often used in combination with penicillins or other antibiotics; see, for instance, L.P. Garrod, H.P. Lambert, F. O'Grady, P.M. Waterworth, "Antibiotic and Chemotherapy", Fifth Edition, Churchill Livingstone, 1981, pages 220 to 225; and EP-A-217 580.

Although fusidic acid has shown some in vitro antiviral effect against certain viruses such as Coxsackie virus A21, and rhinovirus (see, for example, Br. J. Pharmac. Chemother., 1967, 31, pages 210 to 220), it has never been possible to demonstrate a clinical effect of fusidic acid in viral diseases.

It has now, surprisingly, been found that fusidic acid has a clinical effect against AIDS (acquired immunodeficiency syndrome) caused by the retrovirus HIV (human immunodeficiency virus).

Patients suffering from AIDS are susceptible to a number of opportunistic infections which are difficult to treat with usual antimicrobial therapy since the immune system of such patients is damaged and, therefore, does not assist in the elimination of the pathogens. A special problem with AIDS patients is the presence of intracellular microorganisms which cannot be reached by many of the known antibiotics such as β-lactam antibiotics and aminoglycosides.

Since it is known that fusidic acid can penetrate phagocytic cells and kill intracellular bacteria, fusidic acid was administered to AIDS patients with opportunistic infections in the hope that it might assist other antibiotics in combatting these infections. It was, unexpectedly, found that, when fusidic acid was added to the therapeutic regimen of AIDS patients, it caused a remarkable improvement of their general condition which cannot be explained exclusively by the well-known antibacterial effect of the antibiotic. The present invention is based on this unexpected finding.

Thus, the invention is directed to the use of fusidic acid and its pharmaceutically acceptable salts in the manufacture of a medicament for the prophylaxis and/or treatment of AIDS-related complex and full-blown AIDS.

The present invention also relates to a pharmaceutical composition for the prophylaxis and/or treatment of AIDS-related complex and full-blown AIDS, which comprises fusidic acid or a pharmaceutically acceptable salt thereof together with pharmaceutically acceptable, non-toxic excipients.

Furthermore, the invention relates to a method for the prophylactic or therapeutic treatment of AIDS-related complex and full-blown AIDS, which comprises administering an effective amount of fusidic acid or a pharmaceutically acceptable salt thereof to a person in need of such treatment.

The pharmaceutical compositions of the present invention are, generally, prepared by bringing the active ingredient (fusidic acid and/or a pharmaceutically acceptable salt thereof) into association with one or more pharmaceutically acceptable non-toxic excipients.

Specific examples of pharmaceutically acceptable salts are salts of fusidic acid with alkali metals, alkaline earth metals, amines and alkanolamines; for instance, the water-soluble sodium, potassium, ammonium, triethylamine, piperidine, morpholine, cyclohexylamine, monoethanolamine and diethanolamine salts and the slightly water-soluble calcium, magnesium, symdibenzyl-ethylene-diamine, benzyl-β-phenylethylamine and procaine salts. Other suitable salts are, for instance, those with pyrrolidine, piperazine, guanidine, methylamine, ethylamine, benzylamine or similar unsubstituted or substituted amines, furthermore, quaternary amines such as choline and its derivatives, and salts with basic antibiotics such as aminoglycosides.

A pharmaceutically acceptable excipient is to be broadly understood to include any carrier, diluent, filler, binder or other substance such as an inert pharmaceutical additive to facilitate formulation that is acceptable for administration to a human patient from a toxicity viewpoint and that will not substantially interfere with the administration of the active compound.

Depending on the intended mode of administration, the compositions may be in the form of solid, semi-solid or liquid dosage forms such as, for example, tablets, capsules, sterile powders for reconstitution before parenteral administration, suppositories, pills, liquids, suspensions or the like, preferably in unit dosage forms suitable for single administration of precise dosages. Such unit dosage forms preferably contain the active ingredient in an amount of from 0.025 g to 1 g per dosage unit.

For solid compositions, conventional non-toxic solid excipients include, for example, pharmaceutical grades of mannitol, lactose, starch, magnesium stearate, sodium saccharin, talcum, cellulose, glucose, sucrose, magnesium carbonate and the like. The active ingredient as defined above may be formulated as suppositories using, for example, polyalkylene glycols, for example, propylene glycol, as the carrier. Liquid pharmaceutically administerable compositions can, for example, by prepared by dissolving, dispersing etc., an active compound as defined above and optional pharmaceutical adjuvants in a carrier such as, for example, water, saline, aqueous dextrose, glycerol, ethanol and the like to thereby form a solution or suspension. If desired, the pharmaceutical composition to be administered may also contain minor amounts of non-toxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known or will be apparent to those skilled in the art, for example, see Remington's Pharmaceutical Sciences, Mack Publishing Company, Easton, Pennsylvania, 15th Edition, 1975.

For oral administration fast disintegrating formulations are preferred in order to avoid any local irritation which might be caused by fusidic acid at the dosages employed. A particularly preferred formulation of this type is a film-coated tablet which comprises the following components:

Active ingredient 40 to 60 wt.%

Crospovidone 5 to 20 wt.%

Auxiliary agents (e.g., fillers, such as lactose and microcrystalline cellulose; binders, such as gelatine and polyvinylpyrrolidone;
gildants such as silica; lubricants, such as talc and magnesium stearate; disintegrants, preservatives; flavouring agents etc.,) ad 100 wt.%

The film coating, for instance, consists of hydroxypropylmethylcellulose and may contain a colouring agent such as titanium dioxide.

This film-coated tablet is fast disintegrating and shows good bioavailability without any local irritation.

Administration of the active ingredients according to the present invention and of the pharmaceutical compositions described above, can be via any of the accepted modes of administration for antiviral agents. These methods include oral, parenteral, topical and otherwise systemic administration. Parenteral administration is generally characterized by intravenous injection or infusion.

The amount of active ingredient administered will, of course, be dependent on the subject being treated, the severity of the affliction, the manner of administration and the judgement of the prescribing physician. The daily dosage, however, usually varies from 5 to 75 mg kg body weight, preferably 10 to 50 mg/kg body weight, and may, for instance, be given orally in one or more, preferably one to three doses per day or continuously as infusion.

It is advantageous to combine the treatment with the active ingredient of the present invention with other therapy such as antibacterial, antifungal, antiparasitic and/or antiviral drugs.

The following is a typical case history of an AIDS patient treated with fusidic acid.

The patient, a 48 year old male with a past history of hepatitis (HBsAG positive) and recurrent seborrhoic dermatitis was well until 1984 when he consulted a physician since he was suffering from lethargy, fever and weight loss. Upon examination he was found to have generalised lymphadenopathy. He was found to be HIV positive by ELISA and Western blot with a normal T helper lymphocyte count but was allergic to non-specific mitogens. He remained stable with no further deterioration until 1986 when he suffered further weight loss and diarrhoea. Gastro-enterological investigations revealed atrophic gastritis, B12 malabsorption, candidial esophagitis as well as Entamoeba coli, Entamoeba hartmannii, Endolimax nana, Iodamoeba bütschlii and Ascaris lumbricoides in the stool. Mycobacterium hominis was grown from both the stool and the blood following the detection of acid fast bacilli in a lymph node biopsy. The patient was given isoniazid, ethambutol and rifampicin as well as antiparasitic and local antifungal therapy. After six weeks of treatment there was no improvement with respect to his symptoms and he continued to lose weight (16 kg since admission).
At this time, fusidic acid 500 mg t.d.s. (orally) was added to his regimen. Two weeks following commencement he became afebrile, began to put on weight and was able to undergo a transurethral prostectomy, following which he returned to work and has been well since. Two months later he had increased his weight by 10 kg.

in vitro test of fusidic acid against human immunodeficiency virus (HIV)

In view of the favourable clinical results obtained, fusidic acid was tested in vitro against human immunodeficiency virus (HIV) which is the etiologic agent of AIDS.

HIV binds to a specific and selective subset of T-lymphocytes called T-4 helper/inducer cells which are phenotypically defined by the presence of CD4 molecules along their surfaces. The CD4 molecule which is used by cells to recognise antigenes in association with the class I major histocompatibility complex is also the receptor for the virus.

The T-4 cell has the major responsibility for orchestrating virtually all immune functions in humans and, therefore, HIV can do maximal damage to the immune system by eliminating this particular cell.

In the in vitro test for anti-HIV activity, a CD4 positive cell line was used. It was examined which concentrations of fusidic acid were necessary to prevent infection of this cell line with three different retrovirus isolates, namely, CBL-1, ARV-2 and RF.

The results showed that fusidic acid at a concentration of 50 µg/ml is able to inhibit the HIV prototype isolate CBL-1 from infecting the CD4 positive cell line and that 100 µg/ml was required to prevent infection of this cell line with the two other isolates ARV-2 and RF.

Plasma concentrations of the active ingredients of the present invention exceeding 100 µg/ml can easily be attained by, for example, oral or intravenous administration of doses which are essentially non-toxic. Since it is known that fusidic acid concentrations in leucocytes and lymphocytes are 7 to 10 times higher than in the surrounding medium it is, thus, possible to inhibit HIV replication in humans with non-toxic doses.

The following examples serve to illustrate the present invention but should not be construed as narrowing it or limiting its scope in any way.

EXAMPLE 1

Capsule

Sodium fusidate 250 g
Polyvidone 10 g
Magnesium stearate 5 g

Moisten the sodium fusidate with a solution of polyvidone in ethanol methylenechloride (1:9) and dry the granulate at 35 °C. Sieve the granulate through a 0.6 mm sieve, mix with magnesium stearate. Fill the granulate in hard gelatine capsules No. 0 using a fill weight of 265 mg yielding approx. 1000 capsules.

EXAMPLE 2

Tablet

Sodium fusidate 200 g
Polyvidone 10 g
Crospovidone 50 g
Lactose 210 g
Talc 25 g
Magnesium stearate 5 g

Moisten the sodium fusidate with a solution of polyvidone in ethanol methylenechloride (1:9) and dry the granulate at 35 °C. Sieve the granulate through a 1 mm sieve and mix with Crospovidone, lactose, talc and magnesium stearate. Press 1000 tablet of 500 mg each, using a tablet press equipped with 12 mm circular punches.

EXAMPLE 3

Tablet

Sodium fusidate 250 g
Polyvidone 12.5 g
Crospovidone 50 g
Talc 36 g
Colloidal silica 5 g
Microcrystalline cellulose 72 g
Lactose 72 g
Gelatine 0.4 g
Magnesium stearate 2 g
Hydroxypropylmethylcellulose 8 g
Titanium dioxide 0.5 g

Moisten the sodium fusidate with a solution of polyvidone in ethanol methylenechloride (1:9) and dry the granulate at 35 °C. Sieve the granulate through a 1 mm sieve. Granulate the lactose with gelatine dissolved in water, dry at 60 °C and sieve the granulate through a 1 mm sieve. Mix the granulates with the Crospovidone, talc, silica, cellulose and magnesium stearate. Press 1000 tablets of 500 mg each using a tablet press equipped with 7.8 x 16 mm oval punches. Fillm-coat the tablets in a fluid-bed coater with a solution prepared from hydroxypropylmethylcellulose and titanium dioxide in equal parts of ethanol and water.

EXAMPLE 4

Injectable solution

A solution for intravenous injection is prepared by dissolving the following dry substance in the solvent prior to use:

Dry substance

Sodium fusidate 1000 g

Dry-fill 1 g of sodium fusidate asceptically in each sterile vial and close with a rubber stopper and an aluminium cap.

Solvent

| | |
|---|---|
| Citric acid | 20 g |
| Sodium phosphate | 390 g |
| Water for injection | up to 20 litres |

Dissolve the citric acid and sodium phosphate in water for injection.

Filter the solution through a 0.2 µm membrane filter and fill 20 ml into each vial. Close the vials with rubber stoppers and aluminium caps. Finally, sterilize the solution by autoclaving at 121 °C for 15 minutes.

## Claims

1. The use of fusidic acid and its pharmaceutically acceptable salts in the manufacture of a medicament for the prophylaxis and treatment of AIDS-related complex and full-blown AIDS.

2. A composition for the prophylaxis and treatment of AIDS-related complex and full-blown AIDS, which comprises fusidic acid or a pharmaceutically acceptable salt thereof together with pharmaceutically acceptable, non-toxic excipients.

3. A composition according to claim 2 in the form of a sterile powder for reconstitution before parenteral administration.

4. A composition according to claim 2 in the form of an aqueous suspension.

5. A composition according to claim 2 in the form of a tablet or capsule.

6. A composition according to claim 5, containing fusidic acid or a pharmaceutically acceptable salt thereof in an amount of 0.025 g to 1 g per dosage unit.

7. Film-coated comprising the following components in the amounts indicated (based on the total weight):

| | |
|---|---|
| Fusidic acid or a pharmaceutically acceptable salt thereof | 40 to 60 wt.% |
| Crospovidone | 5 to 20 wt.% |
| Auxiliary agents | ad 100 wt.% |

8. Fusidic acid and its pharmaceutically acceptable salts for use in the prophylaxis and treatment of AIDS-related complex and full-blown AIDS.

9. A method of treating AIDS-related complex and full-blown AIDS, which comprises administering an effective amount of fusidic acid or a pharmaceutically acceptable salt thereof to a person in need of such treatment.

10. A method according to claim 9 which comprises the prophylactic treatment of a person in risk of contracting AIDS-related complex and full-blown AIDS.

11. A method according to claim 9 which comprises the therapeutic treatment of a patient suffering from AIDS-related complex and full-blown AIDS.

12. A process for the preparation of a composition according to any one of claims 2 to 7 which comprises bringing fusidic acid or a pharmaceutically acceptable salt thereof into association with one or more pharmaceutically acceptable, non-toxic excipient(s).

European Patent Office

# PARTIAL EUROPEAN SEARCH REPORT

which under Rule 45 of the European Patent Convention shall be considered, for the purposes of subsequent proceedings, as the European search report

Application number
EP 87 11 0621

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP - A - 0 217 580 (THE WELLCOME FOUNDATION)<br>* Claims 24-26,36,37,41,42,9-22 *<br>-- | 1-8,12 | A 61 K 31/71<br>A 61 K 31/56 |
| A | CHEMICAL ABSTRACTS, vol. 74, no. 13, 29th March 1971, abstract no. 73192b<br>Columbus, Ohio, US;<br>T. GONCHARSKAYA et al.: "Antiviral properties of fusidin"<br>& ANTIBIOTIKI (MOSCOW), 1971, 16(1), 57-60<br>* Abstract *<br>-- | 1 | |
| A | CHEMICAL ABSTRACTS, vol. 77, no. 10, 4th September 1972, page 92, abstract no. 70720y<br>Columbus, Ohio, US;<br>S.S. GERASIMOVA et al.: "Effect of Fucidin on the reproduction of Venezuelan equine encephalomyelitis virus in tissue culture"<br>& ANTIBIOTIKI (MOSCOW), 1972, 17(5),457-61<br>* Abstract * -- | 1 | TECHNICAL FIELDS SEARCHED (Int. Cl.4)<br>A 61 K 31/00 |

-2-

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8,12
Claims searched incompletely:
Claims not searched: 9-11
Reason for the limitation of the search:

Method for treatment of the human or animal body by surgery or therapy (see art. 52(4) of the European Patent Convention).

| Place of search | Date of completion of the search | Examiner |
|---|---|---|
| THE HAGUE | 14-03-1988 | ISERT |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application number
EP 87 11 0621
-2-

| DOCUMENTS CONSIDERED TO BE RELEVANT | | | CLASSIFICATION OF THE APPLICATION (Int. Cl 4) |
|---|---|---|---|
| Category | Citation of document with indication, where appropriate, of relevant passages | Relevant to claim | |
| A | CHEMICAL ABSTRACTS, vol. 80, no. 7, 18th February 1974, abstract no. 34077x Columbus, Ohio, US; T.Ya. GONCHARSKAYA et al.: "Effect of fusidin on smallpox vaccine virus", & ANTIBIOTIKI(MOSCOW) 1973, 18(8) 710-13 * Abstract * | 1 | |
| | -- | | TECHNICAL FIELDS SEARCHED (Int. Cl 4) |
| T | THE LANCET, vol. 2, 10th October 1987, pages 827-828 V. FABER et al.: "Inhibition of HIV-replication in vitro by fusidic acid" * Pages 827-828 * | | |

--------------------

EPO Form 1505.3 06.78